Europäisches Patentamt

European Patent Office

Office européen des brevets

(11) Publication number: **0 200 350**
A2

# (12) EUROPEAN PATENT APPLICATION

(21) Application number: 86302194.5

(22) Date of filing: 25.03.86

(51) Int. Cl.⁴: **C 07 H 21/04**

(30) Priority: 15.04.85 US 723306

(43) Date of publication of application:
05.11.86 Bulletin 86/45

(84) Designated Contracting States:
AT BE CH DE FR GB IT LI LU NL SE

(71) Applicant: THE ONTARIO CANCER INSTITUTE
500 Sherbourne Street
Toronto Ontario M4X 1K9(CA)

(72) Inventor: Mak, Tak W.
130 Glen Eoad
Toronto Ontario M4W 2W3(CA)

(74) Representative: Crisp, David Norman et al,
D. YOUNG & CO. 10 Staple Inn
London, WC1V 7RD(GB)

(54) Nucleic Acid encoding a T-cell Antigen Receptor Polypeptide.

(57) A cDNA clone has been isolated which encodes the α chain of the T cell antigen receptor from a human T cell line, Jurkat. This encoded sequence is about 1101 nucleotides. The sequence may be used as a probe to determine whether an unknown cell is a T cell. Polypeptides encoded by the cDNA alone include about 277 amino acid residues comprising the human T cell receptor α chain. Antibody to the polypeptide may be prepared and used to identify T cell antigen receptor and to assay for T cells.

EP 0 200 350 A2

Croydon Printing Company Ltd.

## Nucleic Acid encoding a T-cell Antigen
## Receptor Polypeptide

This invention relates to a nucleic acid encoding a polypeptide which is at least part of the $\alpha$-chain of a T-cell antigen receptor.

T (thymus derived) cells, like B cells, recognize specific antigens. This recognition is essential for activation of T cells, which have at least two major functions in the immune system. They kill cells that appear foreign, such as cells that have been infected with viruses and carry viral antigens and they regulate other immune responses, including antibody production by B cells (H.R. Green et al., (1983) Ann. Rev. Immunol. 1, 439-461; P.C. Kung et al., (1983) Intl. J. Dermatol. 22, 67-76).

Antigen recognition is thought to be mediated through a receptor on T cells. Molecular and biochemical characterization of the T cell antigen receptor should clear up many of the mysteries concerning T cell recognition of antigen and should help in attaining a better understanding of the myriad interactions between the immune cells and their targets and consequently, a better understanding of the regulation of immune responses.

In spite of extensive efforts, progress in characterizing the cell antigen receptor during the past two decades has been slo (J.J. Marchalonis, (1982) Immunol. Today 3, 10-12; M. Kronenberg et al., (1983) Cell. 34, 327-329). Recently severa groups of investigators have, however, produced murine monoclonal antibodies that may recognize T cell surface proteins with the predicted characteristics of the antigen receptor (J.L. Marx, (1983) Science 221, 444-446). These antibodies were produced by immunizing mice with cell lies of cloned T cells; and the antibodies react with only the cells used to elicit the antibody production.  Immunochemical studies in man (S.C. Meuer et al., (1983) J. Exp. Med. 157, 705-719; S.C. Meuer et al., (1983) J. Exp. Med. 158, 988-993; R.D. Bigler et al, (1983) J. Exp. Med. 158, 1000-1005; Acuto, O. et al (1983) Cell. 34, 717-726; J. Kappler et al., (1983) Cell. 35, 295-302; S.C. Meuer et al., (1983) Science 222, 1239-1242) and in rodents (K. Haskins et al., (1983) J. Exp. Med. 157, 1149-1161; P. Marrack et al., (1983) J. Exp. Med. 158, 1635-1646; B.W. McIntyre and J.P. Allison, (1983) Cell 34, 739-746; J. Kappler et al., (1983) Cell. 34, 727-734) suggest that the putative T cell antigen receptor is a molecule composed of two different polypeptide chains, designated the α and β chains, with molecular weights of approximately 40,000 and 45,000 daltons, respectively. Under nonreducing conditions the T cell receptor molecule is a disulfide-linked heterodimer with a molecular weight of 85,000 to 90,000 daltons.  Peptide mapping analysis in the murine system suggests that the T cell receptor protein contains a variable and a constant region. However, these studies have provided no information concerning the protein or nuleotide sequences.

Recently, there have appeared several reports that researchers have prepared cDNA from T cell mRNA which encodes the β chain T cell receptor protein in mice (Hedrick, S. et al. (1984) Nature 308, 153-1588 and humans (Yanagi, Y. et al. (1984) Nature 308, 145-149).

Subsequent studies have indicated that the genes encoding these structures undergo somatic rearrangement in T lymphocytes and are homologous to the immunoglobulin (Ig) genes.  In addition, their genomic structures are similar, but not identical in organization, to Ig genes.  These results indicate that genes encoding the T cell receptor, at least the β- chain, are different from Ig genes, though they most likely share a common ancestor (Mak, T.W. et al. (1984) Immunological Reviews 81,

0200350

221-233). The finding that the human β chain variable and constant region genes are located on chromosome 7, where there are no Ig genes, confirmed that these genes are independent of Ig genes. These observations, coupled with the fact that α and β chains are approximately the same size, suggest that the α-chain is probably also Ig related and may contain similar variable and constant domains.

This invention relates to the isolation and detailed molecular characterization of the human T cell α chain antigen receptor protein and the gene which encodes it. The invention also concerns a method of isolating and screening for the receptor gene, the nucleotide sequence of the gene, the amino acid sequence of the protein encoded by the gene and uses of the nucleotide sequence and the protein including the determination whether an unknown cell is a T cell. This latter aspect may be realized through the production of serum or monoclonal antibodies from the α chain receptor protein or portions thereof.

While these studies were in progress, isolation of the murine α-chain cDNA (Chien, Y. et al (1984) Nature 312, 31-35; Saito, H. et al (1984) Nature 312, 36-40) and partial protein sequence of the human α-chain (Hannum, C. et al. (1984) Nature 308, 153-158; Fabbi, M. et al. (1984) Nature 312, 269-271) were reported. The elucidation of the α and β chains of the T cell receptor should provide the molecular tools necessary for the study of the recognition of antigens and major histocompatibility complex products by human T cells.

Brief Description of the Drawings

Figure 1 shows Northern and Southern gel analysis of non-rearranging cDNA clones pY4, pY17, and pY25;

Figure 2 shows the expression of pY14 in T and non-T cells;

0200350

Figure 3 shows Southern gel analysis of T and non-T cells;

Figure 4 shows the nucleotide deduced amino acid sequences of T cell clone pY14; and

Figure 5 shows a direct comparison of the deduced amino acid sequence of clone pY14 with those of β chain of Jurkat and murine λ light chain.

## MATERIALS AND METHODS

### Cells

Jurkat cell line was first isolated by Schneider et al (1977) Int. J. Cancer 19, 621-626, and is generally available to researchers in this field. All other cell lines used have been described previously (Yanagi, Y. et al. (1984) Nature 308, 145-149; Yoshikai, Y. (1984) Nature 312, 521-524).

### Construction of cDNA Libraries

Double stranded cDNA was generated from poly(A)$^+$ RNA of a human leukemic T cell line, Jurkat, using the approach described previously (Yanagi, Y. et al. ibid). For the subtracted library, double stranded cDNA was tailed using dCTP and terminal deoxynucleotidyl transferase and inserted into PstI site of plasmic PBR322 by C-G annealing. The vector ggt10 was used. EcoR1 linkers were used to ligate double stranded DNA to the ggt10 phage arms. The ggt10.cDNA hybrids were packaged in vitro and plated as described previously (Davis, R.W. et al (1980) A Manual for Genetic Engineering, Advanced Bacterial Genetic, Cold Spring Harbor Lab.).

## Cloning of T cell Specific cDNAs

cDNA copied from poly(A)$^+$ RNA from Jurkat was hydrolysed in 0.1M NaOH to remove poly(A)$^+$ RNA templates and hybridized to excess poly(A)$^+$ RNA from a B cell line, RPMI 1788. Hybridizaton was carried out in 50% formamide and 5 times SSC at 42° to Crt of over 2000. Double stranded cDNA was then separated from the unannealed single stranded T cell "enriched" cDNA with hydroxylapatite columns as described by Britten et al (1974) Methods in Enzymology 29, 363-406. These single stranded cDNA were used to construct a T cell "enriched" cDNA library as described above. This subtracted library was examined by differential screening using single stranded T cell "enriched" cDNA and RPMI 1788 cDNA as probes (Yanagi, Y. et al. ibid).

## Northern Blot Analysis

Northern blot analysis of T cell and non-T cell RNA was carried out according to the procedure of Thomas (1980) Proc. Natl. Acad. Sci. 77, 5201-5205. RNA was extracted from different cells in the presence of guanidine thiocyanate. Poly(A)$^+$ RNA was isolated using oligo dT cellulose column, then treated with glyoxal. Poly(A)$^+$ RNA was electrophoresed through 1% agarose. Transfer of the RNA to nitrocellulose membrane was as described previously (Thomas, ibid). Hybridization was to P$^{32}$ dCTP labelled nick translated DNA fragments.

## Southern Blot Analysis

Southern blot analysis with T cell specific cDNAs or the T cell receptor α chain cDNA was performed using the method described previously (Southern, E.M. (1975) J. Mol. Biol. 38, 503-517). 10 mg of DNA was electrophoresed through 0.8% agarose and transferred to nitrocellulose filters as described by Southern (ibid). Hybridization to $^{32}$P-dCTP labelled nick translated DNA fragment was as described previously (Toyonaga, B. et al. (1984) Nature 311, 385-387).

0200350

DNA Sequencing

For DNA sequencing, the ggt10.Y14 recombinant was digested with EcoR1 and the cDNA insert was purified twice by electrophoresis through 1% low melting point agarose, then further digested with four nucleotide cutter restriction enzymes and ligated into M13mp9. DNA sequencing was performed using the dideoxy chain terminating inhibitor method of Sanger et al. (1977) Proc. Natl. Acad. Sci. USA 74, 5463-5467.

RESULT

Non-rearranging T cell Specific cDNA Clones from the Human Leukemic T cell Line Jurkat

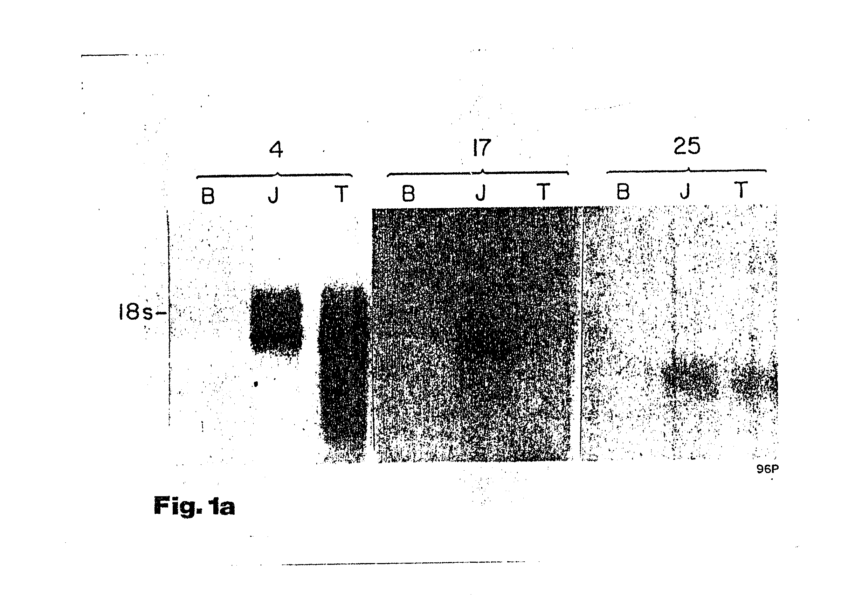

T cell specific cDNAs were obtained by the subtraction method combined with differential hybridization, as described in Materials and Methods. These T cell specific clones were then grouped into 19 different groups on the basis of their cross hybridization to each other and the sizes of messages to which they correspond in T cells or thymocytes. In addition, these clones were analyzed to examine whether they undergo somatic rearrangement in DNA from T cells (leukemic cells) when compared to the corresponding germ line DNA (normal bone marrow cells from the same patient). Using restriction enzymes BamHI, EcoRI and Hind III, Southern blot analysis did not show any gene rearrangement for these T cell specific clones. Some of the messages from which these clones were derived can also be found in thymocytes, while others were found only in Jurkat. Some examples of these studies are illustrated in Fig. 1. Fig. 1a shows Poly(A)$^+$ RNA from (B) B cell line RPMI 1788, (J) T cell line Jurkat and (T) thymocytes was analyzed by Northern gel analysis using cDNA inserts from clone pY4, pY17 and pY25. While mRNA homologous to pY17 can be found only in Jurkat, messages corresponding to pY4 and pY25 can be found in both Jurkat and thymocytes. An example of Southern blot analysis of these T cell specific clones is shown in Figure 1b. DNA was

extracted from T cell leukemia blast cells (L) and normal
fibroblast (N) cultures from the same patient. DNA was
digested with BamHI, Hind III or EcoRI and analysed using cDNA
insert from clone pY25. This figure indicates that using
restriction enzymes BamHI, Hind III and EcoRI, no rearrangement
can be detected for pY25. Table I summarizes the results of
these analyses.

## Table I

### T cell clones from Jurkat cDNA library

| Class | Size of mRNA (kb) | No. of Clones | B | J | T | Rearrangement | Clone Designation |
|-------|-------------------|---------------|---|---|---|---------------|-------------------|
|       |                   |               | Expression | | | | |
| A | 3.0 | 3 | − | + | − | − | |
| B | UD | 3 | | | | − | |
| C | 1.7 | 1 | − | + | + | − | |
| D | 2.0/1.4 | 1 | − | + | + | − | pY4 |
| E | 1.3 | 1 | − | + | − | − | pY17 |
| F | 1.0 | 1 | − | + | + | − | pY25 |
| G | 2.2/2.0/1.0 | 1 | | | | − | |
| H | UD | 1 | | | | − | |
| I | UD | 1 | | | | ? | |
| J | UD | 1 | | | | − | |
| K | UD | 1 | | | | − | |
| L-S | ND | 1 each | | | | − | |
| β chain | 1.3/1.0 | 20 | − | + | + | + | |
| α chain | 1.6/1.3 | 1 | ± | + | + | + | pY14 |

44

UD - undetectable          B: B cell line RPMI 1788

ND - not done              J: Jurkat

? - not clear              T: Thymus

## Expression of Clone pY14

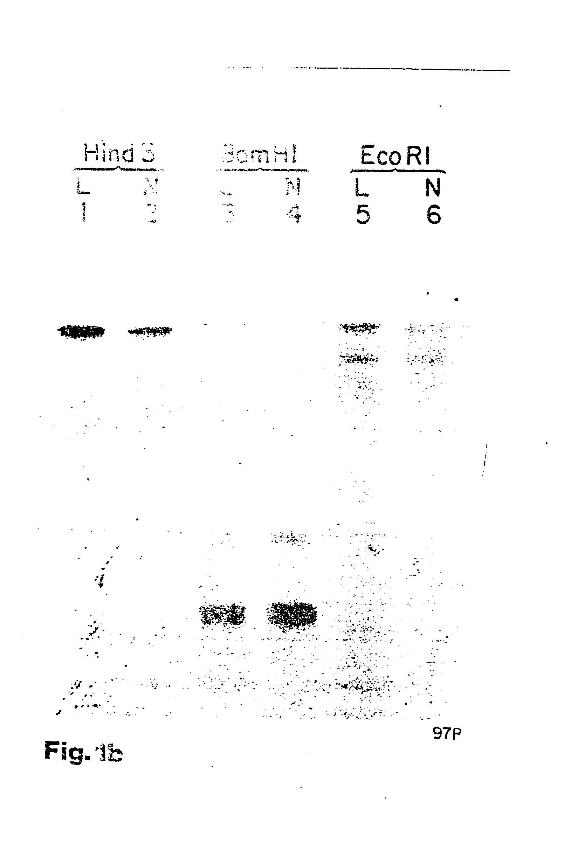

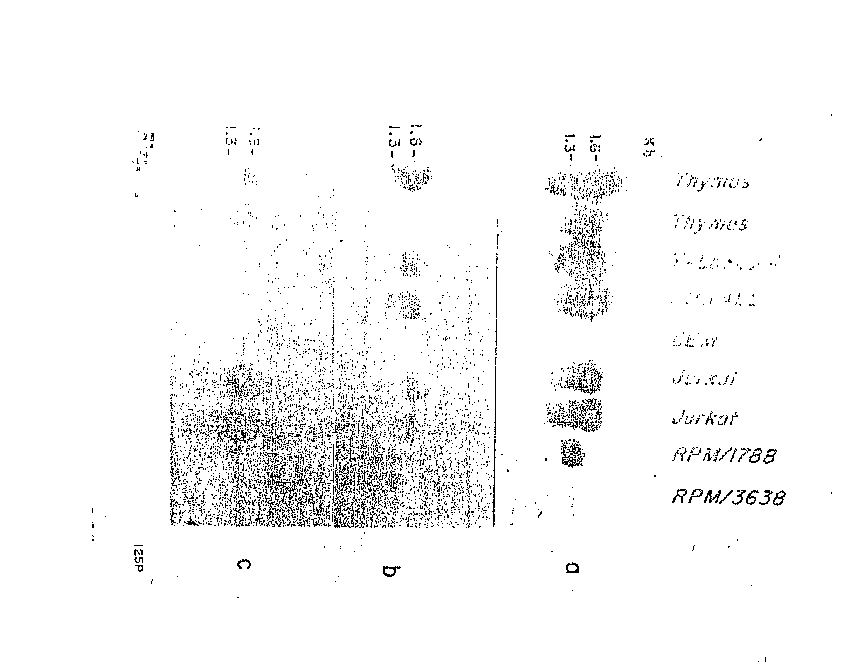

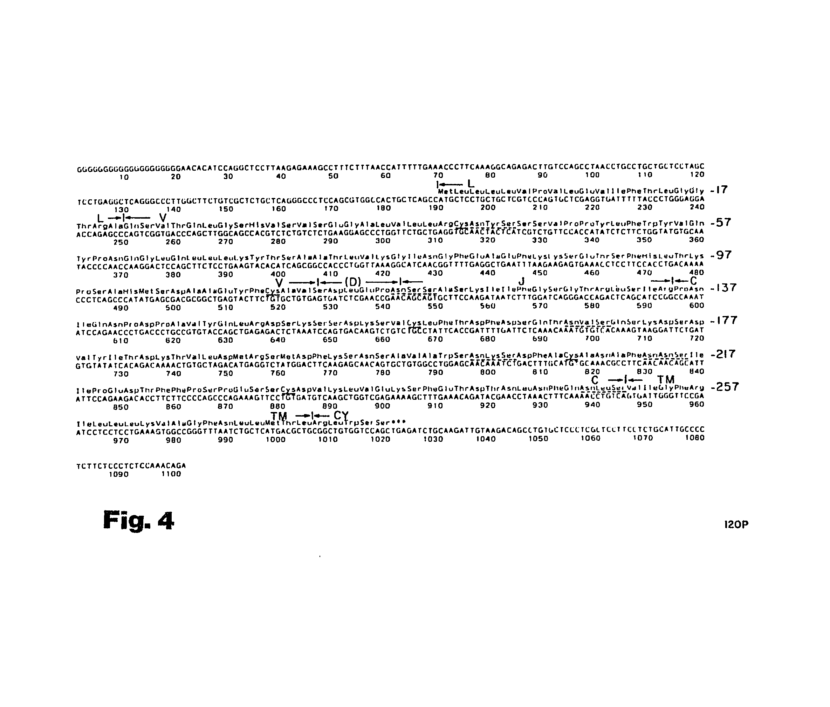

In addition to the non-rearranging T cell specific cDNAs described above, T cell receptor β and α chain cDNAs were also isolated from a T cell (Jurkat) cDNA library. With the use of DNA sequence information (Yoshikai, Y. et al. ibid) 20 β chain cDNAs were identified amongst the T cell specific clones. With oligonucleotide probes corresponding to the mouse α chain, a positive human clone, pY14 was also identified. The expression of clone pY14 is illustrated in Figure 2. Poly(A)$^+$ RNA from T and non T cells was examined using Northern blot analysis. The probes used were (a) total pY14 insert; (b) HpaII-HpaII fragment of pY14 (nucleotides 591 to 980 Figure 4) corresponding to the constant region of the α chain, (c) EcoRI-Sau3A fragment of pY14 (nucleotides 1-529 Figure 4) corresponding to the variable region. Lanes 1 & 2, (from the left) show human thymocytes; 3, T cell leukemic cell; 4, HPB-ALL; 5, CEM; 6 & 7, Jurkat; 8, B cell line RPMI 1788; and 9, B cell line RPMI 3638. RNA was prepared from cells of T cell lineage as well as non-T cell lineages. Northern blot analysis indicated that the human thymocytes from two individuals, blast cells from a patient with T cell leukemia, and two of the three leukemic T cell lines (Jurkat and HPB-ALL) expressed a message of approximately 1.6 kb, corresponding to the pY14 sequence. In addition, a shorter message of about 1.3 kb was observed in HBP-ALL. This short message can also be detected in lower amounts in B cell lines, RPMI 1788 and RPMI 3638. However, pY14 failed to hybridize with RNA from a leukemic T cell line (CEM), a erythroleukemia line (data not shown) and a bladder tumor line (MGHU-1) (data not show). To examine whether the expression of these messages, especially the 1.3 kb message in B cell lines, may be due to the expression of sequences homologous to the variable and or constant region of the DNA pY14, RNA was examined using a 5' end (EcoRI-Sau3A fragment, nucleotides 1-529 in Figure 4) and a 3' end (HpaII-HpaII fragment, nucleotides 591-980) probe of the cDNA. Figure 2b illustrates that all T cells, except for a leukemic T cell line CEM, contain messages homologous to the 3' end of pY14 (constant region, see below). In addition, a shorter message of about 1.3 kb of B cell lines was found to hybridize with the 3' end probe. However, when the 5' end probe was used (variable region, see below), hybridization was observed only to T cell line Jurkat and thymocytes (Figure 2c). Hybridization of two thymocytes samples was considerably weaker for the 5, end probe than for the complete cDNA probe or the 3'end probe. No hybridization was observed to other T or B cell RNAs.

## Somatic Rearrangement of pY14 in T cells

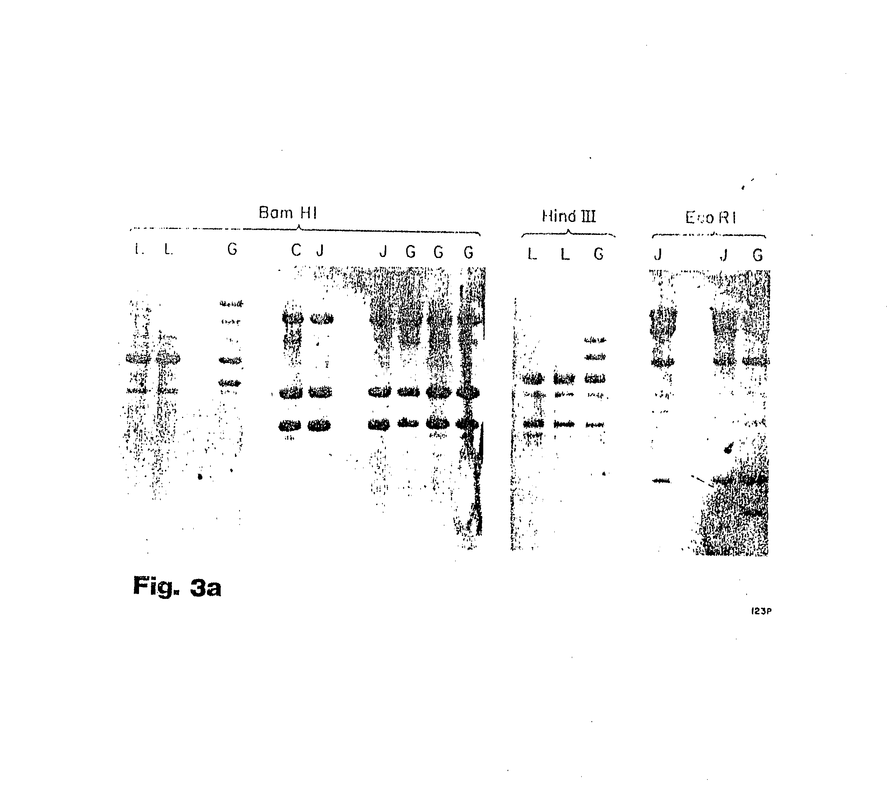

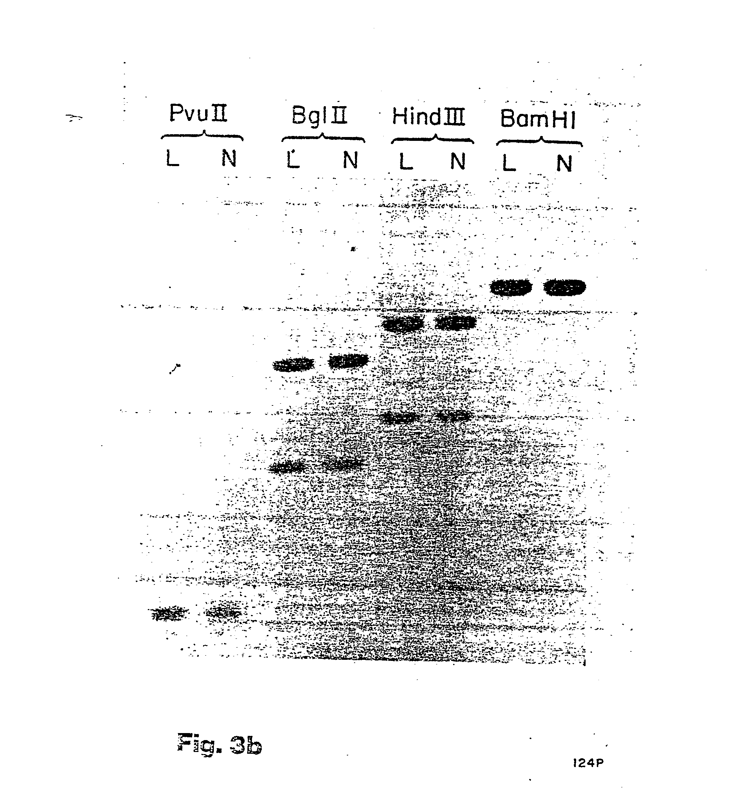

In order to determine the germ line organization of the genes corresponding to pY14 and whether they undergo rearrangement in T cells, DNA from 15 T cells and 4 non-T cells was digested with restriction enzymes: EcoRI, BamHI, HindIII, BglII, PvuII and XhoI. The digested DNA was examined by Southern gel analysis using the complete insert of pY14 was a probe (Figure 3). DNA was analysed by Southern gel analysis using (3a) the complete pY14 insert as a probe; (3b) constant region probe (HpaII-HpaII nucleotides 591-980 Figure 4), where G is germ line; L, T cell leukemic cells; J, Jurkat; and C, T cell line CEM. Although no difference in banding patterns was found in all the non-T cell samples examined, different but varied banding patterns were observed in DNA from several T cell lines and leukemic T cells from patients. For example, with the restriction enzyme BamHI (Figure 3a), 6 bands can be seen in germ line DNA, whereas only 5 germ line fragments in a DNA sample from the T cell line, Jurkat, and 3 bands in a sample from a patient with T cell leukemia can be observed. No apparent gene rearrangement was found for the T cell line, CEM. Similarly, 7 germ line bands were observed using EcoRI while only 5 bands were detected in Jurkat DNA. Another example of rearrangement was seen in DNA from leukemic cells digested with HindIII. To estimate how many of these germ line bands may be variable or constant region fragments and if rearrangement can be found using the constant region probe, we hybridized the BamHI, Hind III, BglII and PvuII digested DNA

from normal and leukemic cells with a constant region probe (the 3' end of pY14). Results in Figure 3b indicate that only 1 or 2 bands can be assigned to the constant region. In addition, no rearrangement can be found using this probe in DNA from a patient with T cell leukemia digested with these restriction enzymes.

Nucleotide and Deduced Protein Sequences of pY14

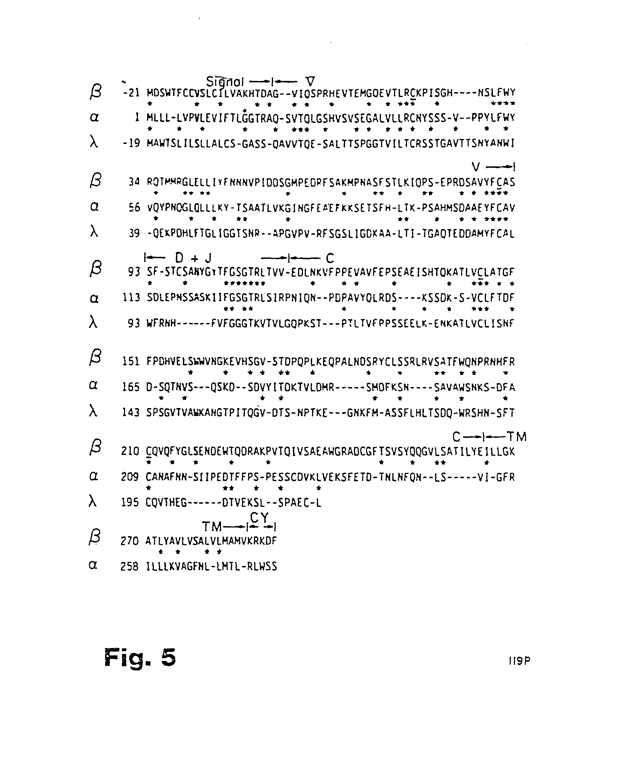

A sequence of 1101 nucleotides of pY14 is shown in Figure 4. The deduced amino acid sequence is derived from the only open reading frame in the nucleotide sequence. The cysteine residues are underlined and the potential N-glycosylation sites are indicated by interrupted underlines ((L), leader sequence; (V), variable; (D), diversity; (J), joining; (C), constant; (TM), transmembrane; and (CY), cytoplasmic). These regions are assigned by comparison with their chain counterparts. The nucleotide sequence has a long open reading frame with the first methionine codon ATG at nucleotide 190 and a TGA termination triplet at position 1021. A protein composed of amino acids of this open reading frame has a molecular weight of 29,995 daltons. There are two stretches of uncharged amino acids, from amino acid 1 to 20 and from 253 to 272. It is possible that the first stretch of non polar amino acids corresponds to a signal peptide while the second stretch resembles a transmembrane anchor, suggesting that this is a transmembrane protein. The protein sequence has regions similar to the signal, variable (V), diversity (D), joining (J) and constant (C) region of Ig and T cell receptor β chain proteins (Figure 5). In Figure 5 identity between the sequences is indicated by an asterisk (*). Gaps (-) were introduced to increase matching. Symbols for the amino acids are the commonly accepted nomenclature. β, represents JUR-β1 corresponding to the β chain of Jurkat; α,represents pY14 corresponding to the α chain; and λ, represents murine λ light chain. Six potential glycosylation sites are identified, with one in the variable region (amino acid 43), one in the J region (amino acid 118), and 4 in the constant region (amino acids 169, 203, 214, an 250). Comparison DNA and protein sequences of pY14 with those of the murine α chain cDNA and the partial protein sequence of the human α chain, indicates that very high homology can be found between pY14 and these sequences, indicating that pY14 encodes the human α chain. This deduced protein sequence shows extensive similarity to the β chain from Jurkat and the murine λ light chain throughout most of its sequence (Figure 5). The highest homology can be found near the cysteine residues at amino acids 42, 110 and 159 of pY14, and within the putative J region. Like the β chain protein, the human α chain contains an additional peptide that extends beyond the C terminus of the murine λ light chain. The overall homology of the α protein to that of the β chain is about 30% while the similarity to the murine λ chain is about 25%.

From the foregoing it should be clear to one of ordinary skill that the nucleic acid sequence of the clone pY14 having at least about 1101 nucleotides, encodes a polypeptide which is at least part of a T cell antigen receptor, e.g., a primate T cell antigen receptor, preferably a human T cell antigen receptor. The nucleic acid may be DNA, including cDNA, or RNA. The invention also includes a nucleic acid having at least about 60% homology with pY14.

The nucleic acid sequences may be used as probes if labeled with a detectable marker, e.g., a radioactive, fluorescent or biotinylated marker. Labeled probe nucleic acid sequences according to the invention may be used, inter alia, to determine whether an unknown cell, e.g., a tumor cell, is a T cell.

Host cells containing a suitable cloning vehicle which includes cDNA encoding the T cell antigen receptor may be prepared and used to produce the receptor polypeptide in accordance with methods known to those of ordinary skill in the art.

The invention also concerns polypeptides encoded by the nucleic acid sequences including a polypeptide which is at least part of a T cell antigen receptor and contains at least about 277 amino acids e.g. as shown in Figure 4.

Antibodies to the polypeptides, e.g., monoclonal or serum-derived antibodies, may be prepared using methods known to those skilled in the art. Such antibodies may be employed to detect the presence of T cell receptor antigen and to determine whether an unknown cell, e.g., a tumor cell, is a T cell. To do so the sample to be tested is treated with the antibody under suitable conditions permitting formation of antigen antibody complex and a determination made whether complex is present. For example, see Towbin, H., et al., Proc. Natl. Acad. Sci. USA 76, 4350 (1979); Monoclonal Antibodies and T Cell Products (ed. Katz, D.H.); Lerner, R.A., Nature 299, 592 (1982); and Lerner, R.A., et al., Proc. Natl. Acad. Sci. USA 77, 5197 (1980).

CLAIMS:

1.      A nucleic acid having a sequence encoding a polypeptide which is at least part of the α chain of a primate T cell antigen receptor, comprising at least 1101 nucleotides.

2.      A nucleic acid as claimed in claim 1, wherein the nucleic acid is DNA.

3.      A nucleic acid as claimed in claim 1, wherein the nucleic acid is RNA.

4.      A nucleic acid as claimed in claim 2 having the nucleotide sequence shown in Figure 4.

5.      A substantially pure DNA being approximately 60% or more homologous with the nucleic acid of claim 4.

6.      A nucleic acid as claimed in claim 1, wherein the T cell is a human T cell.

7.      A nucleic acid as claimed in claim 1 labeled with a detectable marker.

8.      A cloning vehicle containing the nucleic acid of claim 1.

9.      A microorganism containing the cloning vehicle of claim 8.

10.      A polypeptide encoded by a nucleic acid sequence of claim 1.

11.      A polypeptide encoded by a nucleic acid sequence of claim 4.

12.    A polypeptide which is at least part of the α chain of a primate T cell antigen receptor, comprising at least about 277 amino acids.

13.    A polypeptide as claimed in claim 12, wherein the T cell is a human T cell.

14.    A polypeptide as claimed in claim 12, having the sequence of 277 amino acids as shown in Figure 4.

15.    An antibody directed to an antigenic determinant of a polypeptide of claim 12.

16.    An antibody as claimed in claim 15, wherein the antigenic determinant is unique to the human α chain T cell receptor.

17.    An antibody as claimed in claim 15, wherein the antibody is a monoclonal antibody.

4 · 17 · 25

B J T    B J T    B J T

96P

18s—

**Fig. 1a**

| Hind 3 | | BamHI | | EcoRI | |
|---|---|---|---|---|---|
| L | N | | N | L | N |
| 1 | 2 | 3 | 4 | 5 | 6 |

97P

**Fig. 1b**

125P

Fig. 3a

Fig. 3b

124P

GGGGGGGGGGGGGGGGGGGGAACACATCCAGGCTCCTTAAGAGAAAGCCTTTCTTTAACCATTTTTGAAACCCTTCAAAGGCAGAGACTTGTCCAGCCTAACCTGCCTGCTGCTCCTAGC

                                                              |←— L
                                                MetLeuLeuLeuValProValLeuGluValIlePheThrLeuGlyGly -17
TCCTGAGGCTCAGGGCCCTTGGCTTCTGTCGCTCTGCTCAGGGCCCTCCAGCGTGGCCACTGCTCAGCCATGCTCCTGCTGCTCGTCCCAGTGCTCGAGGTGATTTTTACCCTGGGAGGA

L —→|←— V
ThrArgAlaGlnSerValThrGlnLeuGlySerHisValSerValSerGluGlyAlaLeuValLeuLeuArgCysAsnTyrSerSerSerValProProTyrLeuPheTrpTyrValGln -57
ACCAGAGCCCAGTCGGTGACCCAGCTTGGCAGCCACGTCTCTGTCTCTGAAGGAGCCCTGGTTCTGCTGAGGTGCAACTACTCATCGTCTGTTCCACCATATCTCTTCTGGTATGTGCAA

TyrProAsnGlnGlyLeuGlnLeuLeuLeuLysTyrThrSerAlaAlaThrLeuValLysGlyIleAsnGlyPheGluAlaGluPheLysIleSerGluThrSerPheHisLeuThrLys -97
TACCCCAACCAAGGACTCCAGCTTCTCCTGAAGTACACATCAGCGGCCACCCTGGTTAAAGGCATCAACGGGTTTTGAGGCTGAATTTAAGAAGAGTGAAACTCCTTCCACCTGACAAAA

                                    V —→|←— (D) —→|←—                      J                      —→|←—C
ProSerAlaHisMetSerAspAlaAlaGluTyrPheCysAlaValSerAspLeuGluProAsnSerSerAlaSerLysIleIlePheGlySerGlyThrArgLeuSerIleArgProAsn -137
CCCTCAGCCCATATGAGCGACGCGGCTGAGTACTTCTGTGCTGTGAGTGATCTCGAACCGAACAGCAGTGCTTCCAAGATAATCTTTGGATCAGGGACCAGACTCAGCATCCGGCCAAAT

IleGlnAsnProAspProAlaValTyrGlnLeuArgAspSerLysSerSerAspLysSerValCysLeuPheThrAspPheAspSerGlnThrAsnValSerGlnSerLysAspSerAsp -177
ATCCAGAACCCTGACCCTGCCGTGTACCAGCTGAGAGACTCTAAATCCAGTGACAAGTCTGTCTGCCTATTCACCGATTTTGATTCTCAAACAAATGTGTCACAAAGTAAGGATTCTGAT

ValTyrIleThrAspLysThrValLeuAspMetArgSerMetAspPheLysSerAsnSerAlaValAlaTrpSerAsnLysSerAspPheAlaCysAlaAsnAlaPheAsnAsnSerIle -217
GTGTATATCACAGACAAAACTGTGCTAGACATGAGGTCTATGGACTTCAAGAGCAACAGTGCTGTGGCCTGGAGCAACAAATCTGACTTTGCATGTGCAAACGCCTTCAACAACAGCATT

                                                                        C —→|←— TM
IleProGluAspThrPhePheProSerProGluSerSerCysAspValLysLeuValGluLysSerPheGluThrAspThrAsnLeuAsnPheGlnAsnLeuSerValIleGlyPheArg -257
ATTCCAGAAGACACCTTCTTCCCCAGCCCAGAAAGTTCCTGTGATGTCAAGCTGGTCGAGAAAAGCTTTGAAACAGATACGAACCTAAACTTTCAAAACCTGTCAGTGATTGGGTTCCGA

                                                TM —→|←— CY
IleLeuLeuLeuLysValAlaGlyPheAsnLeuLeuMetThrLeuArgLeuTrpSerSer∗∗∗
ATCCTCCTCCTGAAAGTGGCCGGGTTTAATCTGCTCATGACGCTGCGGCTGTGGTCCAGCTGAGATCTGCAAGATTGTAAGACAGCCTGTGCTCCCTCGCTCCTTCCTCTGCATTGCCCC

TCTTCTCCCTCTCCAAACAGA

**Fig. 4**

I20P

```
                 Signal ──|── ∇
β   -21 MDSWTFCCVSLCILVAKHTDAG--VIQSPRHEVTEMGQEVTLRCKPISGH----NSLFWY
          *      *   *    *     *  ***  *    * *  * * *   *    ****
α     1 MLLL-LVPVLEVIFTLGGTRAQ-SVTQLGSHVSVSEGALVLLRCNYSSS-V--PPYLFWY
          *    *   *      *    *  ***  *    * *  * * *   *     * *
λ   -19 MAWTSLILSLLALCS-GASS-QAVVTQE-SALTTSPGGTVILTCRSSTGAVTTSNYANWI

                                                             V ──|
β    34 RQTMMRGLELLIYFNNNVPIDDSGMPEDRFSAKMPNASFSTLKIQPS-EPRDSAVYFCAS
          *     ** **         *          *   **    **    * *  ****
α    56 VQYPNQGLQLLLKY-TSAATLVKGINGFEAEFKKSETSFH-LTK-PSAHMSDAAEYFCAV
          *  *   *   **       *                  **    *   * * ****
λ    39 -QEKPDHLFTGLIGGTSNR--APGVPV-RFSGSLIGDKAA-LTI-TGAQTEDDAMYFCAL

        |── D + J        ──|── C
β    93 SF-STCSANYGTFGSGTRLTVV-EDLNKVFPPEVAVFEPSEAEISHTQKATLVCLATGF
          *    *       ******      *   * *   *        *    *** * *
α   113 SDLEPNSSASKIIFGSGTRLSIRPNIQN--PDPAVYQLRDS----KSSDK-S-VCLFTDF
                      ** **                *             *   ***  *
λ    93 WFRNH------FVFGGGTKVTVLGQPKST---PTLTVFPPSSEELK-ENKATLVCLISNF

β   151 FPDHVELSWWVNGKEVHSGV-STDPQPLKEQPALNDSRYCLSSRLRVSATFWQNPRNHFR
          *    *     * * **         *      *    * **  * *      *
α   165 D-SQTNVS---QSKD--SDVYITQKTVLDMR-----SMDFKSN----SAVAWSNKS-DFA
          * *        **         *         *    *  *    *
λ   143 SPSGVTVAWKANGTPITQGV-DTS-NPTKE---GNKFM-ASSFLHLTSDQ-WRSHN-SFT

                                                    C ──|──TM
β   210 CQVQFYGLSENDEWTQDRAKPVTQIVSAEAWGRADCGFTSVSYQQGVLSATILYEILLGK
          *  *   *    *    *    *   *   *       **   *       *
α   209 CANAFNN-SIIPEDTFFPS-PESSCDVKLVEKSFETD-TNLNFQN--LS-----VI-GFR
          *         **  *  *    *       *   *
λ   195 CQVTHEG------DTVEKSL--SPAEC-L

               TM──|─CY─|
β   270 ATLYAVLVSALVLMAMVKRKDF
          *   *    * *
α   258 ILLLKVAGFNL-LHTL-RLWSS
```

## Fig. 5

119P